# EUROPÄISCHE PATENTSCHRIFT

(11) **EP 1 499 252 B1**
(45) Veröffentlichungstag und Bekanntmachung des Hinweises auf die Patenterteilung: **23.11.2005**
(21) Anmeldenummer: 03740933.1
(22) Anmeldetag: 28.04.2003
(51) Int. Cl.: A61B 18/12

(54) **VORRICHTUNG ZUR ÜBERWACHUNG MEDIZINISCHER GERÄTE**
DEVICE FOR MONITORING MEDICAL EQUIPMENT
DISPOSITIF DE SURVEILLANCE POUR APPAREILS MEDICAUX

(30) Priorität: 26.04.2002 DE 10218894
(43) Veröffentlichungstag der Anmeldung: 26.01.2005
(73) Patentinhaber: Storz Endoskop Produktions GmbH, 78532 Tuttlingen (DE)
(72) Erfinder: DANERS, Felix, CH-8200 Schaffhausen (CH)
(74) Vertreter: Lohr, Georg
(86) Internationale Anmeldenummer: PCT/IB2003/003023
(87) Internationale Veröffentlichungsnummer: WO 2003/090790

(56) Entgegenhaltungen:
- DE-A- 3 003 891
- DE-A- 3 736 712
- DE-A- 3 923 024
- US-A- 4 301 801

## Beschreibung

### Technisches Gebiet

Die Erfindung betrifft ein medizinisches Gerät, insbesondere einen Generator zur Leistungserzeugung für die Hochfrequenzchirurgie.

### Stand der Technik

Bei medizinischen Geräten steht neben der eigentlichen Behandlung die Sicherheit von Patienten und Behandlern im Vordergrund. Viele medizinische Geräte wirken durch elektrische bzw. mechanische Energie auf den Patienten ein bzw. benötigen zur Ausführung ihrer Funktionen in ihrem Inneren elektrische bzw. mechanische Energie.
Durch diese Energieformen kann es bei einer Fehlbedienung bzw. einer Fehlfunktion des Gerätes zur Gefährdung von Patienten bzw. Behandlern kommen. Das Gefährdungspotenzial soll am Beispiel eines Hochfrequenzchirurgiegenerators erläutert werden.
In der Hochfrequenzchirurgie wird menschliches oder tierisches Körpergewebe mittels elektrischem Strom geschnitten bzw. koaguliert. Die Hochfrequenzchirurgie ist insbesondere in Verbindung mit endoskopischen Operationstechniken äußerst vorteilhaft einsetzbar.
Die Aufgabe der Hochfrequenzchirurgiegeneratoren ist es, die elektrische Energie für die Hochfrequenzchirurgie derart bereitzustellen, dass das gewünschte Operationsergebnis erreicht wird. Um Muskel- bzw. Nervenreizungen zu minimieren, liefern Hochfrequenzchirurgiegeneratoren hochfrequente Energie im Frequenzbereich über 300 kHz. Diese hochfrequente Energie wird meist mittels einer Elektrode in das Gewebe eingespeist. Am Punkt der Einspeisung tritt eine starke Erwärmung des die Elektrode umgebenden Gewebes auf. Wird in einem kurzen Zeitintervall eine hohe Energie zugeführt, so führt dies zu einem Verdampfen der Zellflüssigkeit und einem Aufplatzen der Zellen, so dass sich der Zellverband um die Elektrode auflöst. Die Elektrode kann sich nahezu frei durch das Gewebe bewegen. Wird über längere Zeit eine geringere Energie zugeführt, so führt dies zu einer Koagulation des Gewebes d. h. zu einer Gerinnung des Eiweißes. Die Zellen sterben hierbei ab und werden zu einer zähen Masse.
Grundsätzlich werden bezüglich der Einleitung der hochfrequenten Energie zwei Anordnungen unterschieden.

Bei der monopolaren Anordnung wird eine kleinflächige Schneide- bzw. Koagulationselektrode zur Stromeinleitung an Operationsort und eine großflächige "neutrale Elektrode" zur Stromausleitung an einem anderen Ort des Körpers des Patienten angeordnet. Die Elektrodenfläche ist hier so groß dimensioniert, dass es zu keiner nennenswerten Wärmeentwicklung an der Elektrode kommt.

Die bipolare Anordnung umfasst eine zweigeteilte Elektrode, bei der die Stromeinleitung sowie die Ausleitung am Operationsort erfolgt.

Ein Hochfrequenzchirurgiegenerator kann also erhebliche Energiemengen vom Netz beziehen, in seinem Inneren speichern und diese auch über längere Zeit an den Ausgang abgeben, um Gewebe zu schneiden bzw. zu Koagulieren. Es werden hohe Sicherheitsanforderungen an einen Hochfrequenzchirurgiegenerator gestellt, so dass eine unbeabsichtigte Leistungsabgabe an den Ausgang mit größter Zuverlässigkeit vermieden wird. Ebenso sollte auch im ersten Fehlerfall keine höhere als die vom Operateur vorgewählte Leistung abgegeben werden.

So ist in der US-Patentschrift US 6,142,992 eine Leistungsbegrenzung vorgesehen, welche die Leistungsabgabe des Generators automatisch begrenzt, wenn die Elektrode in keinem Kontakt zum Gewebe steht.

In der Patentschrift DE 197 14 972 C2 ist eine Überwachung der Neutralelektrode angegeben. Sie verhindert Bedienungsfehler beim Anlegen der Neutralelektrode.

In der US-Patentschrift US 4,301,801 ist ein Sicherheitsschaltkreis vorgesehen, der bei einer überhöhten Ausgangsspannung des Generators dessen Stromversorgung unterbricht.

Die im Stand der Technik bekannten Sicherheitseinrichtungen beziehen sich auf die Überwachung der äußeren Generatorfunktionen und den Schutz vor Fehlbedienung. Ebenso wichtig wie der Schutz vor äußerer Fehlbedienung ist jedoch ein Schutz vor inneren Fehlfunktionen des Gerätes.

Die meisten heute bekannten medizinische Geräte werden durch einen zentralen Steuerrechner bzw. Mikroprozessor oder auch Controller genannt, gesteuert. Bekannte Verfahren zur Sicherheitsüberprüfung, wie zusätzliche Abfrageprozeduren in der Software des Hauptcontrollers schützen nicht vor einem Ausfall dieses Controllers und auch nicht vor Programmierfehlern. Eine mehrfach redundante Anordnung identischer Controller bedingt einen sehr hohen Materialaufwand und schützt ebenfalls nicht vor Programmierfehlern.

### Darstellung der Erfindung

Der Erfindung liegt die Aufgabe zugrunde, eine Sicherheitsüberwachung von Generatoren für die Hochfrequenzchirurgie bereitzustellen, welche bei höchster Zuverlässigkeit und niedrigen Kosten eine hohe Erkennungswahrscheinlichkeit interner Fehler und auch externer Bedienungsfehler aufweist.

Eine erfindungsgemäße Lösung dieser Aufgabe ist im Patentanspruch 1 angegeben. Weiterbildungen der Erfindung sind Gegenstand der abhängigen Ansprüche.

Die erfindungsgemäße Vorrichtung umfasst einen Hochfrequenzgenerator für die Hochfrequenzchirurgie, welcher Einstellelemente (30), funktionale Elemente (31) und einen Hauptcontroller (6) zur Abfrage der Einstellelemente bzw. Steuerung der funktionalen Elemente aufweist. Die Einstellelemente (30) dienen zu Vorgabe von Werten bzw. zur Auswahl von Funktionen des Gerätes. Sie umfassen alle Elemente, die für die Einstellung bzw. Steuerung von Gerätefunktionen notwendig sind, wie Tasten, Schalter oder auch Elemente zur Rückmeldung wie Anzeigen. Die funktionalen Elemente (31) führen die entsprechenden Funktionen, welche durch die Einstellelemente vorgegeben sind, aus. Der Hauptcontroller (6) dient als zentrales Kommunikations- und Steuerelement und umfasst vorzugsweise einen Mikrocontroller oder einen programmierbaren Logikbaustein. Er ist durch entsprechende Signalisierungsleitungen bzw. Bussysteme mit den Einstellelementen und den funktionalen Elemente verbunden.

Weiterhin ist ein Sicherheitscontroller (20) vorgesehen, welcher Einstellelemente sowie funktionale Elemente abfrägt und mit den gewonnenen Informationen Plausibilitätsprüfungen zur Erkennung von unzulässigen Betriebszuständen bzw. Fehlern durchführt. Weiterhin gibt der Sicherheitscontroller bei unzulässigen Betriebszuständen bzw. im Fehlerfall ein Fehlersignal ab, welches die funktionalen Elemente deaktiviert bzw. in einen sicheren Betriebszustand versetzt. Selbstverständlich können auch mehrere Sicherheitscontroller vorgesehen werden. So können verschiedene Bereiche des Gerätes von verschiedenen Controllern überwacht werden. Ebenso ist es möglich, mehrere Controller parallel als redundantes System für dieselben Überwachungsaufgaben einzusetzen. Weiterhin ist eine kaskadierte Struktur aus Sicherheitscontrollern möglich, bei der ein Controller der nächsten Ebene jeweils Plausibilitätsprüfungen der Funktionen des Controllers der vorhergehenden Ebene durchführt.

Eine solche erfindungsgemäße Anordnung ist wesentlich sicherer und zuverlässiger als die bekannten Anordnungen mit einem zweiten Hauptcontroller. Erfindungsgemäß werden Überprüfungen nicht durch Vergleich mit einem redundanten System, sondern auf einem zweiten, unterschiedlichen und unabhängigen Weg durchgeführt. Sie erfolgen hier beispielsweise durch eine vollständig anders geartete Hardware verbunden mit einer anderen Software, die auf anderen Abläufen und Grundsätzen basiert. Dies reduziert systematische Fehler in Hardware und Software wesentlich.

In einer weiteren Ausgestaltung der Erfindung sind Einstellelemente zu Vorgabe von Werten bzw. zur Auswahl von Funktionen ausschließlich zur Steuerung des Sicherheitscontrollers vorgesehen. Durch eine derartige Trennung der Einstellelemente kann eine Beeinflussung des Sicherheitscontrollers durch andere defekte Bauelemente, insbesondere im Bereich des Hauptcontrollers, ausgeschlossen werden. So kann beispielsweise zur Einstellung bestimmter, insbesondere kritischer Gerätefunktionen die gleichzeitige Bestätigung zweier Schalter bzw. Tasten vorgesehen werden. Hierbei steuert eine erste Taste den Hauptcontroller, während eine zweite Taste dem Sicherheitscontroller das Aktivieren der Funktion signalisiert. Hierbei wird ein unbeabsichtigtes auslösen einer Gerätefunktionen durch versehentliches betätigen einer Taste ausgeschlossen und gleichzeitig eine Beeinflussung des Sicherheitscontrollers durch andere defekte Bauelemente bzw. Baugruppen im Fehlerfall vermieden.

Es wird der Anschaulichkeit halber anstelle des allgemeinen Begriffes des Einstellelementes auf den anschaulicheren Begriff der Taste Bezug genommen.

Ebenso kann eine einzige Taste auch mit zwei getrennten Kontaktelementen zur Ansteuerung des Hauptcontroller und des Sicherheitscontrollers ausgebildet werden.

In einer weiteren vorteilhaften Ausgestaltung der Erfindung sind Messabgriffe bzw. Sensoren, verbunden mit funktionalen Elementen vorgesehen, welche Informationen über den Gerätestatus ausschließlich an den Sicherheitscontroller übertragen. Durch diese Ausgestaltung mit redundanten Messabgriffen bzw. Sensoren können auch Fehler, hervorgerufen durch fehlerhafte Messabgriffe bzw. Sensoren, welche mit dem Hauptcontroller kommunizieren, erkannt werden. Gibt beispielsweise ein Gerät auf Grund einer defekten Ausgangspannungsmessung eine zu hohe Ausgangsspannung ab, welche jedoch dem Hauptcontroller als korrekt signalisiert wird, so würde ein Sicherheitscontroller, welche dieselbe Ausgangspannungsmessung verwendet, ebenfalls einen korrekten Wert annehmen. Durch eine zusätzliche, redundante Ausgangspannungsmessung kann der Sicherheitscontroller jedoch feststellen, dass eine zu hohe Ausgangsspannung abgegeben wird und daraufhin das Gerät deaktivieren.

In einer besonders vorteilhaften Ausgestaltung der Erfindung sind Steuerelemente bzw. Schaltelemente vorgesehen, die es ausschließlich dem Sicherheitscontroller ermöglichen, das gesamte Gerät bzw. funktionale Elemente zu deaktivieren bzw. in ihrem Betrieb zu beeinflussen. Dadurch kann eine Überlagerung unterschiedlicher Steuersignale, wie beispielsweise von einem fehlerhaft funktionierenden Hauptcontroller mit den Signalen eines Sicherheitscontrollers, welcher versucht, das Gerät zu deaktivieren bzw. in einen sicheren Zustand zu bringen, vermieden werden.

Eine weitere vorteilhafte Ausgestaltung der Erfindung sieht eine Vorrangschaltung bei den von Hauptcontroller und Sicherheitscontroller gemeinsam genutzten Steuerelementen bzw. Schaltelementen vor. Diese Vorrangschaltung gibt dem bzw. einem Sicherheitscontroller die höhere Priorität bzw, den Vorrang bei der Steuerung der Steuer- bzw. Schaltelemente.

Eine weitere vorteilhafte Ausgestaltung der Erfindung sieht Mittel zur Signalisierung es Fehlerzustandes vor, mit deren Hilfe der Sicherheitscontroller einen Fehlerzustand bzw. seinen Eingriff in das Gerät angezeigt. Derartige Signalisierungsmittel können beispielsweise ein optisches Anzeigeelement, wie eine Lampe bzw. eine Leuchtdiode, Mittel akustischen Signalisierung, oder auch Mittel zur elektrische Signalisierung mittels Steuersignalen bzw. Kommunikation über Bussysteme sein. Ebenso kann auch eine Anzeige vorgesehen sein, welche durch verschiedene optische Signale, wie Blinksignale oder auch durch Klartext den Fehlerzustand, insbesondere weitere Informationen über den Fehlerzustand signalisiert. So kann beispielsweise auf den Schweregrad des Fehlerzustandes oder auf ausgefallene Baugruppen bzw. funktionale Elemente hingewiesen werden.

In einer weiteren vorteilhaften Ausgestaltung der Erfindung ist eine Telekommunikationseinrichtung vorgesehen, mittels deren der Sicherheitscontroller mindestens einer Zentraleinheit einen Fehlerzustand signalisiert bzw. dieser detaillierte Statusinformationen übermittelt. Durch eine solche Übermittlung eines Fehlerzustandes kann die Zentraleinheit bzw. mehrere eventuell sogar miteinander verbundene Zentraleinheiten Fehlerzustände erfassen und auswerten. Neben statistischen Auswertungen über Gerätezuverlässigkeit und Wartungsintervalle ist auch eine gezielte Steuerung bzw. Beeinflussung des fehlerhaften Gerätes möglich. So kann beispielsweise durch die Zentraleinheit ein Ersatzgerät aktiviert werden. Ebenso kann eine automatische Ersatzteilbestellung bzw. Serviceanforderung ausgelöst werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt in allgemeiner Form schematisch eine erfindungsgemäße Vorrichtung.
Fig. 2 erläutert die Erfindung am Beispiel eines Hochfrequenzchirurgiegenerators.

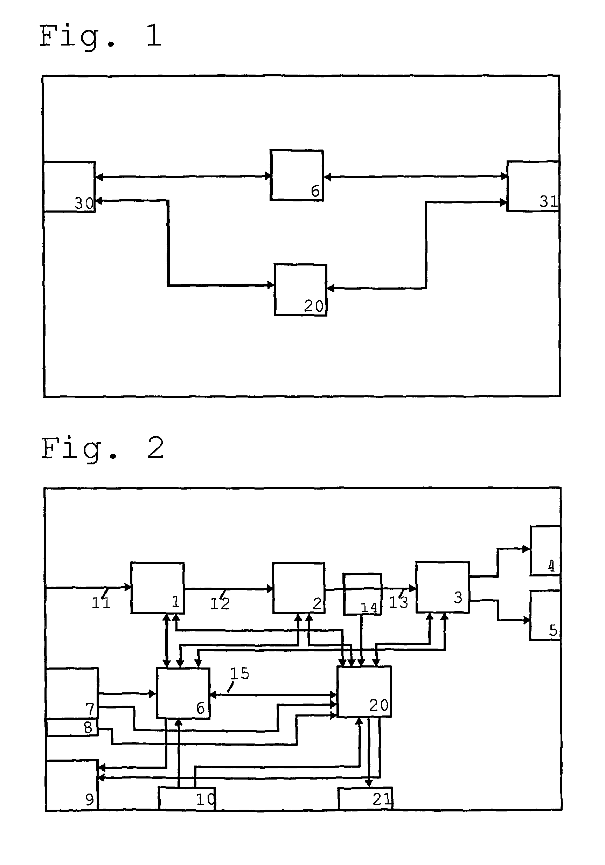

Fig. 1 zeigt in schematischer Form eine erfindungsgemäße Vorrichtung. In einem medizinischen Gerät werden Betriebsparameter durch Einstellelemente (30) vorgegeben. Die Abfrage dieser Einstellelemente erfolgt durch einen Hauptcontroller (6), welcher die funktionalen Elemente (31) entsprechend einstellt und steuert. Weiterhin ist ein Sicherheitscontroller (20) vorhanden, welcher ebenfalls die Parameter der Einstellelemente abfrägt und aus diesen zusammen mit den gemessenen Werten der funktionalen Elemente eine Plausibilitätsüberprüfung durchführt. Im Fehlerfall wird durch den Sicherheitscontroller das System in einen sicheren Betriebszustand überführt bzw. abgeschaltet. Der Sicherheitscontroller kann wahlweise unabhängig vom Hauptcontroller arbeiten bzw. wahlweise zusätzliche Informationen, insbesondere über die korrekte Funktion des Hauptcontrollers von diesem abfragen.

In Fig. 2 ist die Erfindung anschaulich anhand eines Hochfrequenzenergiegenerators dargestellt. Ein Hochfrequenzchirurgiegenerator umfasse ein Netzteil (1), welches aus der Netzspannung (11) einen Leistungsgenerator (2) mit einer Zwischenkreisspannung (12) versorgt. Der Leistungsgenerator selbst gibt eine hochfrequente Spannung (13) ab. Eine Schaltermatrix (3) stellt die Verbindung zwischen dem Leistungsgenerator und beispielsweise einer ersten Ausgangsbuchse (4) zum Anschluss einer bipolaren Elektrode sowie einer zweiten Ausgangsbuchse (5) zum Anschluss einer unipolaren Elektrode und einer Neutralelektrode entsprechend der gewünschten Operationsart her. Ein Hauptcontroller (6) steuert die Funktionen von Netzteil (1), Leistungsgenerator (2) und Schaltermatrix (3). Er bezieht die Statusinformationen von Messabgriffen in Netzteil und Leistungsgenerator. Als Stellelemente zur Auswahl der Betriebsart bzw. Einstellung von Generatorparametern dient eine Tastatur (7), welche eine zusätzliche Freigabetaste (8) für die Hochfrequenzenergie umfasst. Zur Bedienerführung bzw. zur Anzeige von Betriebszuständen ist eine Anzeigeeinheit (9) vorgesehen. Ein zusätzlicher Fußschalter, der über den Fußschalteranschluss (10) anschließbar ist, dient zusätzlich zur Aktivierung des Generators.
Ein Sicherheitscontroller (20) überwacht nun die Eingabesignale der Tastatur (7) um festzustellen, welche Betriebsart bzw. Betriebsparameter eingestellt sind. Zur Abfrage der Tastatur gibt es grundsätzlich mehrere Möglichkeiten. Moderne Hochfrequenzchirurgiegeneratoren benötigen nur sehr wenige Eingabeelemente, so dass die Tasten einzeln jeweils von den Hauptcontroller und dem Sicherheitscontroller abgefragt werden können. Ebenso wäre eine zeitlich versetzte Abfrage der Tasten durch die beiden Controller sinnvoll. Wird ein Tastaturcontroller zur Tastaturabfrage eingesetzt, so übermittelt dieser seine Ausgangsignale sowohl an den Hauptcontroller als auch an den Sicherheitscontroller. In diesem Falle kann der Sicherheitscontroller zusätzliche Sicherheitsüberwachungen, wie z. B. Watchdog- Timer des Tastaturcontrollers vornehmen. Ebenso kann selbstverständlich auch ein kombinierter Tastatur- und Anzeige-Controller eingesetzt werden. Aus den Informationen der Tasteneingaben kann der Sicherheitscontroller nun Rückschlüsse über den gewünschten Betriebszustand des Generators ziehen. Weiterhin kann der Sicherheitscontroller wahlweise bzw. zusätzlich die Anzeigen der Anzeigeeinheit (9) überwachen, um beispielsweise aktuell eingestellte Parameterwerte abzufragen.

Weiterhin bezieht der Sicherheitscontroller von den funktionalen Elementen Netzteil (1), Leistungsgenerator (2) und Schaltermatrix (3) Informationen über deren Betriebszustände. Als zusätzliche Sicherheitsmaßnahmen kann der Sicherheitscontroller durch einen unabhängigen Messabgriff (14) zur Messung der Hochfrequenzspannung die aktuelle hochfrequente Spannung des Generators ermitteln. Weiterhin besteht eine zusätzliche Kommunikationsleitung (15) zwischen dem Sicherheitscontroller und den Hauptcontroller. Mittels dieser kann der Sicherheitscontroller weitere Informationen über Betriebszustände des Generators und das Funktionieren des Hauptcontrollers erhalten.

Der Sicherheitscontroller selbst besteht je nach Komplexität des Generators vorzugsweise aus einem Mikrocontroller oder einem programmierbaren Logikbaustein. Durch Plausibilitätskontrollen im Sicherheitscontroller werden Fehlerzustände des gesamten Systems erkannt.
Da der Sicherheitscontroller nur einfache Plausibilitätsprüfungen durchführt und nicht alle Funktionen des Hauptcontrollers enthält, kann eine Plausibilitätskontrolle selbstverständlich nur im Rahmen der verfügbaren Informationen durchgeführt werden.
Wird beispielsweise eine Operationsart mit konstanter Ausgangsspannung gewählt, so überprüft der Sicherheitscontroller, ob die tatsächlich abgegebene Ausgangsspannung bzw. die durch den Messabgriff (14) angezeigte Ausgangsspannung innerhalb eines vorgegebenen Toleranzbereiches um die gewünschte Ausgangsspannung liegt. Ebenso werden andere Betriebsarten wie beispielsweise konstanter Strom oder konstante Leistung überprüft. Bei komplexeren Betriebsarten, wie Schnitten mit Lichtbogenregelung oder auch Koagulationen mit gepulsten Signalen kann der Sicherheitscontroller das Ausgangssignal auf die Einhaltung von Rahmengrenzwerten, wie z. B. einer maximalen Leistung oder einer maximalen Spannung überwachen. Diese Rahmengrenzwerte können abhängig von der gewählten Operationsart sowie der gewählten Intensität (Lichtbogengröße, Koagulationstiefe, etc.) vorgegeben werden. So kann eine Übermittlung dieser Rahmengrenzwerte je nach Einstellung vom Hauptcontroller an der Sicherheitscontroller erfolgen. Wesentlich besser ist es jedoch, einen Speicher zur Vorgabe dieser Rahmengrenzwerte bzw. einen Berechnungsalgorithmus zur Berechnung der Rahmengrenzwerte aus den aktuellen Einstellungen im Sicherheitscontroller selbst vorzusehen.

Unabhängig von der Einhaltung der Rahmengrenzwerte erfolgt eine Überwachung der aktuellen Betriebszustände. So darf eine Aktivierung des Leistungsgenerators nur dann erfolgen, wenn zuvor die Freigabetaste (8) betätigt wurde und gleichzeitig mit der Aktivierung des Generators der Fußschalter betätigt wird. Ein Fehlerzustand liegt also vor, wenn eine Hochfrequenzspannung am Generatorausgang gemessen wird, ohne dass zuvor die Freigabetaste an der Tastatur betätigt wurde und gleichzeitig mit der Spannungsabgabe der Fußschalter betätigt ist. Weiterhin kann auch das Ausbleiben von regelmäßigen Statusmeldungen des Hauptcontrollers an den Sicherheitscontroller als Fehlerzustand erkannt werden.

Wurde ein Fehlerzustand erkannt, so kann der Sicherheitscontroller in die funktionalen Elemente eingreifen. So kann beispielsweise das Netzteil (1) in der Spannung bzw. im Strom begrenzt oder auch vollständig abgeschaltet werden. Der Leistungsgenerator (2) kann in der Leistungsabgabe durch Taktung reduziert oder ebenso vollständig abgeschaltet werden. Die Schaltermatrix (3) kann so eingestellt werden, dass sie eine Verbindung zwischen Leistungsgenerator (2) und den Ausgangsbuchsen (4,5) auftrennt, sodass eine sichere Trennung zum Patientenstromkreis gegeben ist. Ist können wahlweise einzelne dieser Maßnahmen oder auch alle Maßnahmen gleichzeitig eingeleitet werden.

Die Anzeige von Fehlerzuständen erfolgt beispielsweise über eine Fehleranzeige (21) oder über die Anzeigeeinheit (9) zur Anzeige der Eingaben bzw. Betriebszustände. Die Fehleranzeige ist bevorzugt optisch und kann neben der Tatsache des Fehlers selbst auch detaillierte Auskünfte zur Art des Fehlers abgeben. Dies ist insbesondere dann sinnvoll, wenn der Fehlers leicht durch den Anwender zu beheben ist. Fehlt beispielsweise eine Neutralelektrode, oder ist diese fehlerhaft angeschlossen, so kann der Bediener durch diesen Hinweis dazu veranlasst werden, diesen Fehler zu beseitigen. Bei internen Fehlern ist es wünschenswert dem Servicepersonal konkrete Hinweise zu Fehlerursachen zu geben. Eine zusätzliche akustische Anzeige kann bei besonderen Fehlern warnen.

Werden Fehler erkannt, so können entsprechend dem Fehler Maßnahmen eingeleitet werden. So kann bei einem einfachen Fehler, wie er durch eine Fehlbedienung auftreten kann, eine Abschaltung des Generators und ein Hinweis im Display an den Benutzer erfolgen. Ebenso könnte bei einer kurzzeitigen Überspannungsabgabe während eines koagulierenden Schnittes, der aus zeitlich aufeinanderfolgenden Schnitten und Koagulation besteht, durch den Sicherheitscontroller eine Spannungsbegrenzung erfolgen. Bei schwerwiegenden Fehlern erfolgt eine vollständige Abschaltung des Generators, welche bei besonders schwerwiegenden Fehlern nur noch durch Servicepersonal rückgängig zu machen ist.

Die Aktionen des Sicherheitscontrollers, um das medizinische Geräte in einer sicheren Zustand zu bringen, lassen sich am Beispiel eines Hochfrequenzchirurgiegenerators besonders einfach darstellen, da hier ein sicherer Zustand meist in der Abschaltung des Gerätes besteht. Besonders bei lebenserhaltenden Systemen ist eine Abschaltung des gesamten Systems nicht akzeptierbar. Hier muss das gesamte System bzw. die betroffenen Teilsysteme in ein sicheren Betriebszustand übergeführt werden. Derartige Zustände sind grundsätzlich auch der Hochfrequenzchirurgiegeneratoren denkbar. Wird beispielsweise der Generator gerade während der Koagulation eines größeren Blutgefäßes aufgrund einer Sicherheitsabschaltung vollständig abgeschaltet, so kann dies zu starken und gefährlichen Blutungen des Patienten führen. Für derartige Fälle kann eine manuelle Not-Betriebsart des Generators vorgesehen werden, welche ein Abschließen der Operation ermöglicht. Voraussetzung hierfür ist selbstverständlich, dass der gesamte Hochfrequenz-Leistungspfad noch korrekt arbeitet. So könnte diese Not-Betriebsart beispielsweise beim Ausfall der Lichtbogenregelung oder eines oder mehrerer der hierzu benötigten Komponenten, wie beispielsweise ein Klirrgraddetektor noch verwendet werden.

Weiterhin ist Vorteilhafterweise ein Speicher zur Speicherung der Fehlerzustände im Sicherheitscontroller vorgesehen. So können später aus diesem Speicher Informationen über Art und Rahmenbedingungen der Fehler ausgelesen werden. Besonders zweckmäßig ist es diesen Speicher oder zumindest einen Teil des Speichers als auswechselbares Medium, z. B. als Speicherkarte zu gestalten, sodass diese einfach mit einem entsprechenden Auswertegerät ausgewertet werden kann.

### Bezugszeichenliste

- 1: Netzteil
- 2: Leistungsgenerator
- 3: Schaltermatrix
- 4: erste Ausgangsbuchse
- 5: zweite Ausgangsbuchse
- 6: Hauptcontroller
- 7: Tastatur
- 8: Freigabetaste
- 9: Anzeigeeinheit
- 10: Fußschalteranschluss
- 11: Netzspannung
- 12: Zwischenkreisspannung
- 13: Hochfrequenzspannung
- 14: Messabgriffe
- 15: Kommunikationsleitung
- 20: Sicherheitscontroller
- 21: Fehleranzeige
- 30: Einstellelemente
- 31: Funktionale Elemente

## Patentansprüche

1. Hochfrequenzgenerator für die Hochfrequenzchirurgie umfassend
- Einstellelemente (30) zur Vorgabe von Werten bzw. Auswahl von Funktionen sowie
- funktionale Elemente (31), welche die von den Einstellelementen vorgegebene bzw. zusätzliche fest vorgegebene Funktionen ausführen, und
- mindestens einen Hauptcontroller (6) zur Abfrage der Einstellelemente bzw. Steuerung der funktionalen Elemente
**dadurch gekennzeichnet, dass**
mindestens ein Sicherheitscontroller (20) vorgesehen ist, welcher ebenfalls die Einstellelemente abfragt sowie Statusinformationen der funktionalen Elemente bzw. des Controllers abfragt und mit den gewonnenen Informationen Plausibilitätsprüfungen durchführt, wobei dieser bei unzulässigen Zuständen mindestens ein Fehlersignal abgibt, welches die funktionalen Elemente deaktiviert bzw. in einen sicheren Betriebszustand versetzt.

2. Hochfrequenzgenerator nach Anspruch 1,
**dadurch gekennzeichnet, dass**
Einstellelemente zur Vorgabe von Werten bzw. Auswahl von Funktionen ausschließlich mit dem Sicherheitscontroller (20) verbunden sind.

3. Hochfrequenzgenerator nach Anspruch 1 bzw. 2,
**dadurch gekennzeichnet, dass**
Messabgriffe bzw. Sensoren (14), verbunden mit funktionalen Elementen vorgesehen sind, welche Informationen über den Gerätestatus ausschließlich an den Sicherheitscontroller (20) übertragen.

4. Hochfrequenzgenerator nach einem der Ansprüche 1 bis 3,
**dadurch gekennzeichnet, dass**
Steuerelemente bzw. Schaltelemente ausschließlich für die Steuerung durch den Sicherheitscontroller (20) vorgesehen sind, mit denen das gesamte Gerät bzw. funktionale Elemente deaktiviert bzw. in ihrem Betrieb beeinflusst werden können.

5. Hochfrequenzgenerator nach einem der Ansprüche 1 bis 3,
**dadurch gekennzeichnet, dass**
Steuerelemente bzw. Schaltelemente, welche gemeinsam von Hauptcontroller (6) bzw. Sicherheitscontroller (20) angesteuert werden, eine Vorrangschaltung aufweisen, welche den Sicherheitscontroller die höhere Priorität der Steuerung gibt.

6. Hochfrequenzgenerator nach einem der Ansprüche 1 bis 5,
**dadurch gekennzeichnet, dass**
Mittel zur Signalisierung (21) vorgesehen sind, mit denen der Sicherheitscontroller einen Fehlerzustand beziehungsweise seinen Eingriff in das Gerät anzeigt.

7. Hochfrequenzgenerator nach einem der Ansprüche 1 bis 6,
**dadurch gekennzeichnet, dass**
Mittel zur Telekommunikation mit einer von dem medizinischen Gerät entfernten Zentraleinheit vorgesehen sind, mittels deren der Zentraleinheit ein Fehlerzustand signalisierbar ist und wahlweise detaillierte Zustandinformationen sowie zusätzliche Steuerinformationen bzw. -abfragen von bzw. zu der Zentraleinheit übertragen werden können.

## Claims

1. High-frequency generator for high-frequency surgery, comprising:
- setting elements (30) for presetting values or selecting functions;
- functional elements (31) for performing the functions as preset by the setting elements, and any additional fixed preset functions; and
- at least one main controller (6) for interrogating the setting elements or controlling the functional elements,
**characterized in that**
at least one safety controller (20) is provided for also interrogating the setting elements, and for performing interrogations concerning status information on the functional elements or the controller, and for performing plausibility checks with the obtained information, the safety controller issuing at least one error signal in case of inadmissible conditions, and the error signal deactivating, or putting into a safe working condition, the functional elements.

2. High-frequency generator according to claim 1,
**characterized in that**
setting elements for presetting values or selecting functions are connected exclusively to the safety controller (20).

3. High-frequency generator according to claim 1 or 2,
**characterized in that**
sampling taps or sensors (14) connected to functional elements are provided for transmitting information concerning the instrument status exclusively to the safety controller (20).

4. High-frequency generator according to any one of claims 1 to 3,
**characterized in that**
control elements or circuit elements are provided which are exclusively controlled by the safety controller (20), and which can deactivate, or affect the operation of, the entire instrument or functional elements.

5. High-frequency generator according to any one of claims 1 to 3,
**characterized in that**
control elements or circuit elements that are controlled jointly by the main controller (6) and the safety controller (20) comprise a priority circuit that assigns a higher priority of control to the safety controller.

6. High-frequency generator according to any one of claims 1 to 5,
**characterized in that**
means for signalling (21) are provided, with which the safety controller indicates an error condition or its intervention with the instrument.

7. High-frequency generator according to any one of claims 1 to 6,
**characterized in that**
means for telecommunication with a central unit disposed at a distance from a medical instrument are provided for signalling an error condition to the central unit, and optionally transmitting detailed status information and also additional control information or control interrogations from or to the central unit.

## Revendications

1. Oscillateur haute fréquence pour la chirurgie à haute fréquence, comprenant
- des éléments de réglage (30) pour la détermination des valeurs ou respectivement pour la sélection des fonctions, ainsi que
- des éléments fonctionnels (31) qui réalisent les fonctions prédéterminées par lesdits éléments de réglage ou respectivement des fonctions supplémentaires et prédéterminées d'une manière invariable, et
- au moins un contrôleur principal (6) pour l'interrogation desdits éléments de réglage ou respectivement pour le contrôle desdits éléments fonctionnels,
**caractérisé en ce**
**qu'**au moins un contrôleur de sécurité (20) y est compris, qui appelle également lesdits éléments de réglage ainsi que des informations relatives à l'état desdits éléments fonctionnels ou respectivement dudit contrôleur, et qui réalise des contrôles de vraisemblance moyennant les informations ainsi obtenues, en émettant au moins un signal d'erreur au cas des états inadmissibles, qui désactive lesdits éléments fonctionnels ou respectivement les passe en un état opérationnel sûr.

2. Oscillateur haute fréquence selon la revendication 1,
**caractérisé en ce**
**que** des éléments de réglage sont reliés audit contrôleur de sécurité (20) exclusivement pour la prédétermination des valeurs ou respectivement la sélection des fonctions.

3. Oscillateur haute fréquence selon la revendication 1 ou 2,
**caractérisé en ce**
**que** des prises de mesure ou respectivement des détecteurs (14), reliés auxdits éléments fonctionnels, sont compris, qui transfèrent des informations relatives à l'état du dispositif exclusivement audit contrôleur de sécurité (20).

4. Oscillateur haute fréquence selon une quelconque des revendications 1 à 3,
**caractérisé en ce**
**que** des éléments de commande ou respectivement commutateurs sont disposés exclusivement pour la commande par ledit contrôleur de sécurité (20), moyennant lesquels il est possible de désactiver ou respectivement de prendre de l'influence sur le fonctionnement de tout le dispositif ou respectivement desdits éléments fonctionnels.

5. Oscillateur haute fréquence selon une quelconque des revendications 1 à 3,
**caractérisé en ce**
**que** des éléments de commande ou respectivement commutateurs, qui sont commandés en commun par ledit contrôleur principal (6) ou respectivement ledit contrôleur de sécurité (20), comprennent un circuit de priorité, qui affecte la plus haute priorité en commande audit contrôleur de sécurité.

6. Oscillateur haute fréquence selon une quelconque des revendications 1 à 5,
**caractérisé en ce**
**que** des moyens sont compris pour la signalisation, moyennant lesquels ledit contrôleur de sécurité signalise un état en erreur ou respectivement son intervention dans le dispositif.

7. Oscillateur haute fréquence selon une quelconque des revendications 1 à 6,
**caractérisé en ce**
**que** des moyens de télécommunication comprenant une unité centrale éloignée de l'appareil médical sont disposés, moyennant lesquels il est possible de signaliser à ladite unité centrale un état en erreur et de transférer des informations ou respectivement interrogations de commande supplémentaires à partir ou respectivement à ladite unité centrale.
